# EUROPEAN PATENT APPLICATION

(11) **EP 0 739 613 A1**
(43) Date of publication of application: **30.10.1996**
(21) Application number: 96400844.5
(22) Date of filing: 19.04.1996
(51) Int. Cl.: A61F 2/34

(54) **Acetabulum cup for a total prosthesis of the hip**

(30) Priority: 27.04.1995 FR 9505060
(71) Applicant: BENOIST GIRARD & Cie, 14200 Herouville Saint Clair (FR); Nourissat, Christian, F-42308 Roanne Cédex (FR); Gueret, Alain, F-88000 Epinal (FR); Adrey, José, F-34000 Montpellier (FR); Goalard, Christian, F-34000 Montpellier (FR); Berteaux, Daniel, 45140 Saint Jean de la Ruelle (FR); Hamon, Georges, 59300 Valenciennes (FR); Walter, William, Mosman 2088 (AU)
(72) Inventor: Nourissat, Christian, 42300 Roanne (FR); Gueret, Alain, 88000 Epinal (FR); Adrey, José, 34000 Montpellier (FR); Goalard, Christian, 34000 Montpellier (FR); Berteaux, Daniel, 45140 Saint Jean de la Ruelle (FR); Hamon, Georges, 59300 Valenciennes (FR); Walter, William, Mosman 2088 (AU)
(74) Representative: Rinuy, Santarelli

(57) **Abstract**

The invention relates to an acetabulum cup for a total prosthesis of the hip.

The prosthesis (1) in the form of a spherical cap comprises threaded blind holes (4, 4') on its convex outside surface (3).

The entirely smooth inside surface (2) prevents the premature wear of an insert made of plastic material interposed between it and the head of the femoral part of the prosthesis.

## Description

The present invention relates to an acetabulum cup for a total prosthesis of the hip.

The total prostheses of the hip known at present comprise a femoral prosthetic part, provided with a rod at its distal end and with a spherical head at its proximal end, and an acetabulum cup, serving as a cotyle and having a spherical cavity in which the head of the prosthesis can rotate freely.

The prosthetic acetabulum cups of these known prostheses are attached in to the hip bone of the patient by means of screws or spikes which traverse right through the walls of the cup.

The heads of these screws or spikes used for attaching the cup are generally positioned in recesses accessible from the concave interior of the cup in order to facilite their attachment, after the surgeon has positioned the cup in the patient's acetabulum.

In order to prevent any unwanted contact between the concave inside surface of the acetabulum cup and the spherical head of the prostheses, both of which are made from metallic or ceramic material, there is most often provided an insert made of plastic material in the form of a spherical cup.

This insert is interposed between the acetabulum cup and the head of the femoral element of the prosthesis in order to protect the latter from any direct contact with the concave inside metal surface of the cup and with the heads of the attachment screws or spikes which are located there.

Such a structure, described in particular in European Patent N° 0 404 680, lodged on 27 December 1990 by the applicant, has given excellent results.

Experience has shown, however, that these polyethylene inserts wear during the activities of patients fitted with the prostheses and that the polyethylene debris, due to premature wear, could be the cause of subsequent loosening.

The purpose of the present invention is to overcome these disadvantages.

The acetabulum cup for a total prosthesis of the hip according to the invention is in the form of a spherical cap, comprising two concentric surfaces, inside and outside, defining between them a wall, and it is characterized in that the convex outside surface comprises threaded blind holes having an axial length shorter than the thickness of the wall.

Each of the blind holes can be provided with an externally threaded spike force fitted from the outside. The spikes are tightened up until they reach the bottoms of the blind holes.

Such a cup has the advantage of having a completely smooth concave inside surface.

After milling the cotyloid cavity, to the same diameter as the external diameter of the cup according to the invention, the latter, thus fitted with all of its threaded spikes, is impacted directly into the cavity by the surgeon.

The inside of the cup, thus put into position, can receive a conventional insert made of plastic material or can even be placed in direct contact with the spherical head of the femoral prosthetic part.

The absence of any bore emerging on the inside of the cup prevents any unwanted wear of the element with which the spherical cavity of the cup is called upon to cooperate.

The axes of the blind holes preferably intersect the axis of the cup and can pass through the centre of the sphere defined by the spherical cap.

The orifices through which the blind holes emerge can form two rows, in which they are each distributed symmetrically on the outside surface of the cup, in a staggered arrangement with respect to each other, the axes of the holes of the first row forming, with the axis of the cup, an angle which is different from the one formed by the axes of those of the second row.

According to a particular embodiment, the acetabulum cup according to the invention can be hemispherical.

The present invention will now be described with reference to the accompanying drawings in which:

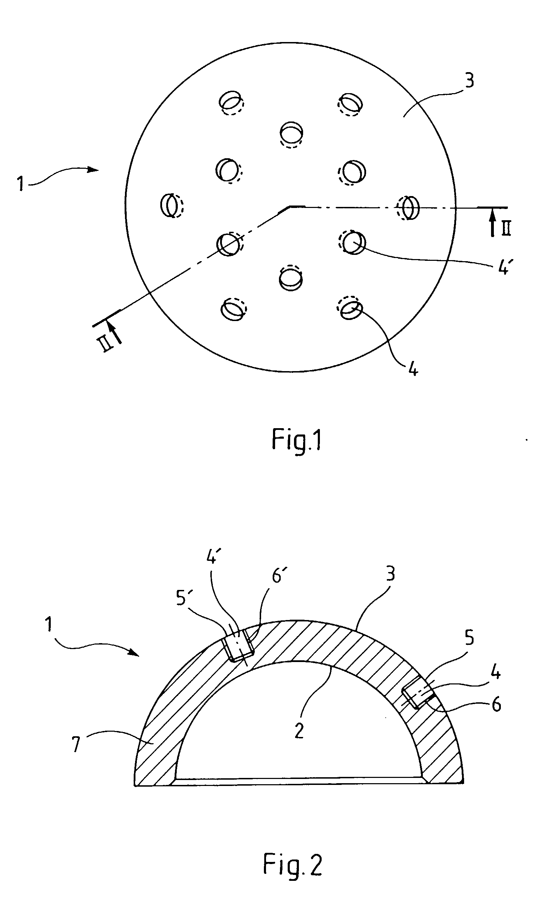

- Figure 1 is a top view of a cup according to the invention,
- Figure 2 is a cross-section along the dotted and dashed line 2-2 of the cup shown in Figure 1,

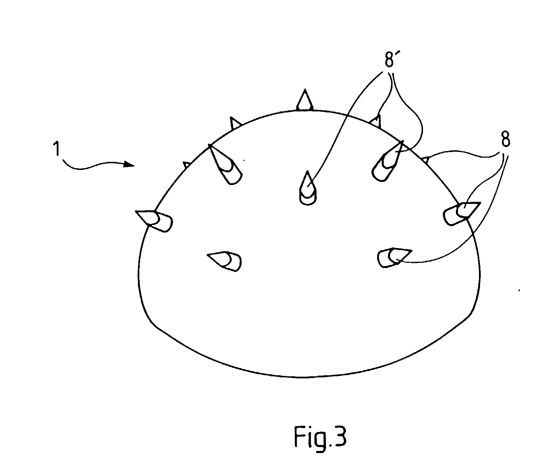

- Figure 3 is a perspective view of the cup shown in the preceding figures and fitted with all of its spikes.

The acetabulum cup according to the invention, indicated by the reference 1, appears in the form of a spherical cap having an inside surface 2 and a concentric outside surface 3.

In the preferred embodiment, shown in the figures, the cup 1 has two rows of blind holes 4 and 4', emerging on the outside surface 3 through orifices 5 and 5', distributed symmetrically around two concentric circles in a staggered arrangement.

Each blind hole has an internal thread 6 and 6' and an axial length shorter than the thickness of the wall 7 of the cup.

As shown in the figures, the angle formed by the axes of the first row of blind holes 4 with the axis of the cup is about 55° and that formed by the axes of the second row is about 30°.

Each blind hole can be fitted with an externally threaded detachable spike 8 and 8' which is screwed by force, from the outside, into each of the blind holes 4 and 4'.

The cup according to the invention, fitted with all of the spikes necessary for its implantation, can then be implanted by the surgeon directly in the patient's acetabulum.

This implantation is facilitated by the staggered arrangement of the spikes in two circular rows.

The implanted cup has an entirely smooth concave inside surface 2 which can cooperate with an insert made of plastic material of complementary shape.

This insert acts as a bearing to ensure universal rotation of the spherical head of the femoral prosthetic part with minimum friction.

Whilst the cup is generally made of a metal material compatible with the bone material, such as titanium and its alloys, the insert is advantageously made from a plastic material known for its low coefficient of friction such as polyethylene or polypropylene.

The absence of any bore emerging on the inside surface of the cup and of any screws or spikes, screwed from the inside of the cup and whose heads would rub against the insert, allows the cup according to the invention to avoid the formation of debris caused by the wear of the insert made of plastic material, which is prejudicial to the stability of the prosthesis and can necessitate its replacement.

## Claims

1. Acetabulum cup (1) for a total prosthesis of the hip, in the form of a spherical cap and having concentric surfaces, inside (2) and outside (3), defining between them a wall (7), characterized in that the convex outside surface comprises threaded blind holes (4, 4') having an axial length shorter than the thickness of the wall.

2. Acetabulum cup according to Claim 1, characterized in that the axes of the threaded blind holes (4, 4') intersect the axis of the cup.

3. Acetabulum cup according to Claim 2, characterized in that the orifices (5, 5') through which the threaded blind holes emerge form two rows, each distributed symmetrically on the outside surface (3) of the cup in a staggered arrangement with respect to each other, the axes of the holes (4) of the first row forming, with the axis of the cup, an angle which is different from the one formed by the axes of the holes (4') of the second row.

4. Acetabulum cup according to any one of the preceding claims, characterized in that the axis of each one of the threaded blind holes (4, 4') passes through the centre of the sphere defined by the spherical cap.

5. Acetabulum cup according to any one of the preceding claims, characterized in that the spherical cap is practically hemispherical.

6. Acetabulum cup according to any one of the preceding claims, characterized in that one or more threaded spikes (8, 8') are force fitted into the various threaded blind holes.
